# EUROPEAN PATENT SPECIFICATION

(11) **EP 2 956 549 B1**
(45) Date of publication and mention of the grant of the patent: **29.04.2020**
(21) Application number: 14705394.6
(22) Date of filing: 12.02.2014
(51) Int. Cl.: C12Q 1/68

(54) **ASSAY FOR DETECTING A NUCLEIC ACID ANALYTE IN A BIOLOGICAL SAMPLE**
VERFAHREN ZUR DETEKTION EINES NUKLEINSÄUREANALYTEN IN EINER BIOLOGISCHEN PROBE
DOSAGE POUR DÉTECTER UN ANALYTE D'ACIDE NUCLÉIQUE DANS UN ÉCHANTILLON BIOLOGIQUE

(30) Priority: 14.02.2013 GB 201302610
(43) Date of publication of application: 23.12.2015
(73) Proprietor: Eluceda Limited, Billington Road Burnley Lancashire BB11 5UB (GB)
(72) Inventor: EASTWOOD, Ian, Rossendale Lancashire BB4 9JZ (GB); CORBALLY, Adam, York Yorkshire YO10 3AJ (GB)
(74) Representative: Murgitroyd & Company
(86) International application number: PCT/GB2014/050401
(87) International publication number: WO 2014/125267

(56) References cited:
- WO-A1-2005/042785
- WO-A1-2008/097190
- JAHIR OROZCO ET AL: "Evaluation of probe orientation and effect of the digoxigenin-enzymatic label in a sandwich hybridization format to develop toxic algae biosensors", HARMFUL ALGAE, ELSEVIER, AMSTERDAM, NL, vol. 10, no. 5, 21 March 2011 (2011-03-21) , pages 489-494, XP028385802, ISSN: 1568-9883, DOI: 10.1016/J.HAL.2011.03.004 [retrieved on 2011-03-29]
- JIAN WANG ET AL: "Electrochemical detection of the neomycin phosphotransferase gene (NPT-II) in transgenic plants with a novel DNA biosensor", JOURNAL OF APPLIED ELECTROCHEMISTRY, KLUWER ACADEMIC PUBLISHERS, DO, vol. 39, no. 6, 14 January 2009 (2009-01-14), pages 935-945, XP019678006, ISSN: 1572-8838
- LIAO J C ET AL: "Use of electrochemical DNA biosensors for rapid molecular identification of uropathogens in clinical urine specimens", JOURNAL OF CLINICAL MICROBIOLOGY, AMERICAN SOCIETY FOR MICROBIOLOGY, US, vol. 44, no. 2, 1 February 2006 (2006-02-01), pages 561-570, XP002581208, ISSN: 0095-1137

## Description

### Field of the Invention

The invention relates to an assay for detecting an analyte in a biological sample. In particular, it relates to an assay for detecting specific nucleic acid sequences or an organism or pathogen in a sample, by measuring potential difference. The invention also relates to a method for detecting specific nucleic acid sequences or an organism or pathogen in a sample and to a kit of parts for use in the method.

### Background to the Invention

A consequence of modern travel means that is almost impossible to contain epidemics at borders. The most recent example of this is the 2009 pandemic of H1N1 swine flu, but there are concerns from transmission of avian flu or a multitude of other pathogens that would use either DNA or RNA to encode the pathogenicity. It takes several weeks or months to develop a test for such a disease. This invention allows for the rapid development of a genotypic test for such an organism, followed by the ability to test individuals for that disease quickly and cost effectively. Such a test could be performed at border control posts clinics, surgeries and pharmacies or even by members of the public who might consider themselves at risk from such an infection. Thus helping to control the spread of the disease from people entering the country or people who might be exposed to the pathogen.

The basic steps of the test are:-
1) Isolation of the pathogen
2) Whole genome sequencing
3) Bioinformatic analysis to identify crucial sequences
4) Generation of target bridging sequences and probe sequence
5) Confirmation of test functionality

Technology that rapidly detects specific DNA sequences has important applications in a wide range of fields from clinical medicine to experimental biology and research diagnostics. A number of approaches have been developed over the past few years. These include optical, acoustic and electronic "gene detection" approaches.

Electronic detection of specifically bound molecules has significant advantages over many currently used systems. In particular, potentiometric systems can provide fast, direct results from the presence of the molecules that are to be detected.

Potentiometric detection involves measuring a potential difference between two electrodes to determine the presence of a specifically bound molecule.

This can be performed by incorporating a stacking system using an oxidising enzyme (such as horseradish peroxidase (HRP), bound to the electrode in the presence of its substrate (H₂O₂).

The enzyme will break down the H₂O₂ into water and oxygen using the electrons from a substrate such as TMB (3,3',5,5'-Tetramethylbenzidine), the compound can be regenerated by accepting electrons from an electrode. In doing so, a potential difference between two electrodes can be generated. This creates a potential difference between the working electrode, which has the enzyme attached to it, and the clean, reference electrode. This is measured as a change in voltage.

Various approaches to the stacking system, which specifically bind the indicator enzyme to the target DNA sequence, have been developed. The aim of such arrangements is to provide a robust specific link between the electrode surface and the indicator enzyme.

One DNA detection system that can be used to detect specific nucleotide sequences involves securely attaching single stranded genomic DNA to the surface of an electrode. A sequence specific biotinylated oligonucleotide is then hybridised to the immobilised genomic DNA. An indicator enzyme such as HRP is linked to the biotinylated oligonucleotide, such that the presence of the indicator enzyme on the electrode is dependent on the presence of the biotinylated oligonucleotide's complementary DNA sequence being present in the genomic DNA on the surface of the electrode Wang et al (J. Appl. Electrochem (2009) 39 935-945) describes an electrochemical detection method using a DNA biosensor involving catalase as indicator enzyme and cyclic voltammetry for detecting the redox reaction.

Blocking and washing strategies are employed to ensure that the biotinylated probe will not bind to the surface of the electrode if the complementary sequence is not present. Without biotinylated probe to bind to neither the streptavidin nor the biotinylated indicator enzyme can attach to the electrode. Therefore an electronic signal will only be detected when the genomic DNA containing the correct sequence is attached to the surface of the electrode.

Standard PCR techniques require two probes a forward and a reverse primer, these are located on different strands of the DNA typically within 1000bp of each other. Although it may be possible to use primers located up to 12kb apart, this procedure is difficult to achieve and requires highly trained technicians and the products of such a PCR would take a long time to produce (1kb per minute x 30 cycles = ∼6 hours) furthermore the products of such reactions are typically contaminated with nonspecific sequences making identification sample DNA difficult.

Some multiplex PCR techniques allow for the detection of multiple DNA sequences and so utilise multiple probes. However, these reactions are extremely difficult to execute properly and require highly trained staff. If the results of a multiplex PCR are to be resolved on a gel then the number of PCR products - and hence probes - are severely limited. Additionally, careful consideration regarding the Tm of each probe also has to be taken into account, as all probes must have a similar melting temperature.

Standard PCR techniques are limited to two probes - and the method of amplification is not analogous to the present DNA bridge method. In PCR, the amplification of the signal is generated by the number of copies created, which in turn is linked to the number of PCR cycles. This means that many PCR cycles are required to amplify a DNA sample to levels visible on an agarose gel. Each cycle has a denaturing stage in which the strands of the chromosome are separated (typically 30 seconds), a hybridisation stage during which the probes hybridise to the template DNA (typically 2 minutes) and an extension phase where the PCR product is created (dependant on the size of the expected PCR product - 1min per kb). Usually 30 cycles are employed, bringing a typical PCR test time to around 2 hours (not including sample collection, cell lysis, DNA extraction and gel analysis). Contaminants at any stage of the PCR reaction can cause test failure furthermore all reagents must be kept cold and frequently go off.

It would be desirable to provide an improved assay for detecting an analyte in a biological sample.

### Summary of the Invention

The scope of the invention is defined by the appended claims.

The disclosure provides an assay for detecting an analyte in a biological sample comprising:
at least one surface probe linked to the surface of a measuring electrode,
the surface probe comprising a nucleic acid sequence complementary to a first target nucleic acid sequence;
at least one signal probe comprising a nucleic acid sequence complementary to a second target nucleic acid sequence;
and wherein the at least one signal probe comprises a binding element capable of binding a ligand,
the ligand being associated with a catalytic element or precursor thereof capable of reacting with a substrate to alter electrical potential of the measuring electrode.

Preferably, the at least one signal probe is substantially free in a test solution when in an unhybridised state.

Advantageously, the at least one signal probe is immobilised by hybridisation to the second target nucleic acid sequence of a single stranded nucleic acid that also comprises the first target nucleic acid sequence.

Advantageously, the at least one signal probe is immobilised by the single stranded nucleic acid tethering the at least one signal probe to the at least one surface probe.

This links the at least one signal probe to the measuring electrode via a genomic DNA or chromosome "bridge" from the sample. This provides the advantage that the genomic DNA from the sample actually forms a "bridge" that tethers the signal probe to the surface probe, and thus to the electrode

The at least one signal probe may comprise a biotinylated oligonucleotide.

Alternatively, other known binding protein systems may be used with the signal probe to link it to a ligand.

The assay preferably, further comprises a reference electrode against which a potential difference between the electrodes can be measured.

The catalytic element may comprise catalase, horseradish peroxidase or a precursor thereof.

In a preferred embodiment, the catalytic element comprises catalase and the substrate comprises hydrogen peroxide.

In one embodiment, the binding element comprises biotin.

The ligand may comprise avidin or streptavidin.

Advantageously, the assay comprises a single hybridisation cycle.

Optionally, the distance between the first target nucleic acid sequence and the second target nucleic acid sequence is greater than 12kB.

In one embodiment, the distance between the first target nucleic acid sequence and the second target nucleic acid sequence may be up to about 50kb.

In one embodiment, the distance between the first target nucleic acid sequence and the second target nucleic acid sequence may be up to about 45kb.

The assay may comprise a plurality of surface probes, signal probes and/or measuring electrodes.

Another aspect of the disclosure provides a method for detecting an analyte comprising the steps of:
providing a single stranded nucleic acid from a sample;
exposing the single stranded nucleic acid to at least one surface probe and at least one signal probe under hybridisation conditions;
   wherein the at least one surface probe is linked to the surface of a measuring electrode, and wherein the at least one signal probe comprises a binding element capable of binding a ligand;
   providing a ligand and a catalytic element associated with the ligand;
   providing a substrate for the catalytic element;
   detecting the presence or absence of an analyte by measuring a potential difference between the measuring electrode and a reference electrode.

Preferably, the at least one signal probe is substantially free in a test solution when in an unhybridised state.

Advantageously, the at least one signal probe is immobilised by hybridisation to the second target nucleic acid sequence when the single stranded nucleic acid from the sample also comprises the first target nucleic acid sequence.

The method may further comprise at least one washing step.

The method may further comprise at least one blocking step.

In a preferred embodiment, the catalytic element comprises catalase.

Preferably, the method comprises the step of adjusting the pH to a pH above or below 7. Preferably the step of adjusting the pH comprises adjusting the pH to between 8 and 9 or between 5 and 6.

In a preferred embodiment, the method comprises the step of increasing the temperature to above about 25°C.

The temperature may be increased to above about 25°C to about 45°C.

Another aspect of the disclosure provides apparatus for detecting an analyte in a biological sample comprising a measuring electrode extending at least partially into a hybridisation chamber,
the hybridisation chamber comprising at least one surface probe linked to the surface of the measuring electrode;
the surface probe comprising a nucleic acid sequence complementary to a first target nucleic acid sequence;
at least one signal probe comprising a nucleic acid sequence complementary to a second target nucleic acid sequence;
wherein the at least one signal probe comprises a binding element capable of binding a ligand, and wherein the ligand is associated with a catalytic element or precursor thereof capable of reacting with a substrate to alter electrical potential of the measuring electrode.

Preferably, the apparatus further comprises a nucleic acid lysis chamber.

Preferably, the apparatus further comprises a filter for filtering DNA.

Yet another aspect of the disclosure provides a kit of parts comprising:
at least one surface probe for linking to the surface of a measuring electrode;
the surface probe comprising a nucleic acid sequence complementary to a first target nucleic acid sequence;
at least one signal probe comprising a nucleic acid sequence complementary to a second target nucleic acid sequence;
a binding element capable of binding a ligand,
a catalytic element or precursor thereof capable of reacting with a substrate to alter electrical potential of the measuring electrode.

On one embodiment, the kit of parts further comprises potentiometric measuring apparatus.

The potentiometric measuring apparatus may comprise at least one measuring electrode and at least one reference electrode.

In one embodiment, the kit of parts further comprises the substrate for the catalytic element.

The disclosure provides an assay that allows the rapid identification of two or multiple DNA sequences from a sample that can be defined following whole genome sequencing and bioinformatic analysis or other means. These DNA sequences may originate from a single chromosome or from different species, depending on how the system is set up. The distance between the two probes can be greater or less than 12kb, and up to 100Mbases. By using the DNA bridging technique of the invention, a method of constructing a high amplification stack within the space of one hybridisation cycle is achieved. By combining this stack with potentiometric enzyme detection techniques and a rapid lysis methodology the assay can produce a "yes/no" result within 10 minutes of sample receipt, at an equal or greater sensitivity than PCR.

The DNA bridging technique allows the identification of multiple sequences that could be as far as 30-50 megabases away from the sequence complementary to that bound to the electrode. This allows the utilisation of pairs of sequences that could not be used in PCR, giving the test/assay of the invention greater flexibility and specificity than other methods.

The DNA bridging technique also allows for the attachment of multiple biotinylated probe sequences to the DNA attached to the electrode.

The signal strength obtained from a sample is proportional to the number of enzymes attached to the DNA. By using multiple probes, this signal can be greatly increased, by providing more points on the "bridge" to which enzyme can attach.

The present system requires a single denaturing step and single hybridisation step allowing for a total test time of a fraction of that of standard PCR.

The present system has no limit to the number of probes that can be used - although it is likely that in practise, only sequences unique to the organism that is to be tested will be used. Despite this, it is possible to use the present system with upwards of a thousand biotinylated probes. This would give an amplification rate of 1- 1000 allowing for extreme sensitivity with a single hybridisation test.

As the assay requires only a single hybridisation step, with no extension phase, the invention provides the advantage of using a temperature gradient during the annealing phase. This will drop from the temperature of the probes with the highest Tm (melting temperature) to that of the lowest Tm allowing binding of all of the probes to the DNA.

This allows a user to be much less fastidious regarding probe Tms when compared to that required when setting up multiplex PCR test and as a result gives a much greater range of target sequences to choose from.

It is possible to use multiple electrodes with the system and multiple probe DNAs (one for each electrode) such that it is possible to detect several elements simultaneously for a pathogen, or multiple organisms. For example, if a pathogen or group of pathogens has two or more pairs of linked sites or loci on a chromosome that is unique to those pathogens - but only the combination of pairs is unique to that organism, then a single surface probe assay would be able to identify that unique organism from the two or more pairs of matching loci.

This could not be achieved using PCR. Other systems using for example antibodies to the cells are susceptible to changes in the antigens on the cell, and are typically less sensitive, in addition antibodies tend to be less effective in the presence of biological materials such as body fluids or serum. Moreover, it is often very difficult to differentiate between the antigenic phenotypes of very similarly related bacteria which can only really be done from the genotype.

One advantage of the disclosure is that hybridisation of nucleotide components occurs in a single step.

Another advantage is that the assay allows the rapid identification of two or multiple DNA sequences from a sample.

The sample chromosomal DNA itself may be used as part of the assay "stack".

This provides a high amplification stack within the space of one hybridisation cycle.

The environmental conditions of the signalling stage can be manipulated to amplify the signal per enzyme. These conditions include pH and temperature of the reaction buffer.

The assay can detect as few as 10 enzymes linked to the surface of the electrode.

Another advantage is that the invention provides a modular assay structure allowing a user to adapt the system to quickly change pathogen targets.

Advantageously, the assay may use a hybridisation stage set on a cooling gradient allowing utilisation of signal and surface probes with different melting points.

Different surface probes comprising of different sequences can be attached to the same electrode. This is advantageous if a test requires looking for the presence of one of a number of pathogens or species in a single test.

Multiple electrodes can be used.

There are also multiple options for covalent binding of the surface probe to the electrode, as will be appreciated by the skilled person.

The assay can detect the presence of enzyme at the surface of an electrode by potentiastatic or amperiometric detection.

Some further advantages of the assay are that it can provide quantifiable results with regards to chromosome numbers and can determine the distance from the surface probe to the signal probe via electronic signal allowing for identification of chromosome type.

Unlike PCR, the assay of the invention does not increase copies of genetic sequence from that found in the sample.

### Brief Description of the Figures

In the Figures, which illustrate embodiment of the invention by way of example only:
Figure 1 is a schematic representation of one embodiment of the assay of the invention.
Figure 2 shows a schematic representation of one embodiment of the potentiometric sensor of the invention.
Figure 3 shows comparative images of electrodes exposed to E.Coli DNA (left) and MRSA DNA (right).
Figure 4 shows a graph illustrating average potential differences against time for *E.Coli* DNA positive and negative samples.
Figure 5 is a bar chart showing total potential differences for *E.Coli* DNA positive and negative samples.
Figure 6 shows a graph illustrating results obtained when using signal probes at different distances from the surface probe.
Figure 7 shows a graph illustrating the results obtained when using a single signal vs multiple signal probes.
Figure 8 shows a graph illustrating the results obtained from electrodes with and without catalase in 100mM Tris pH 5, 7, and 9.
Figure 9 shows a graph illustrating the results obtained from electrodes with catalase minus blanks in 100mM Tris pH 5, 7, and 9.
Figure 10 shows a graph illustrating the results obtained from electrodes with catalase at different reading solution temperatures.
Figure 11 shows a PCR gel image of PCRs run with lysed cell extract.

### Detailed Description of the Preferred Embodiments of the disclosure

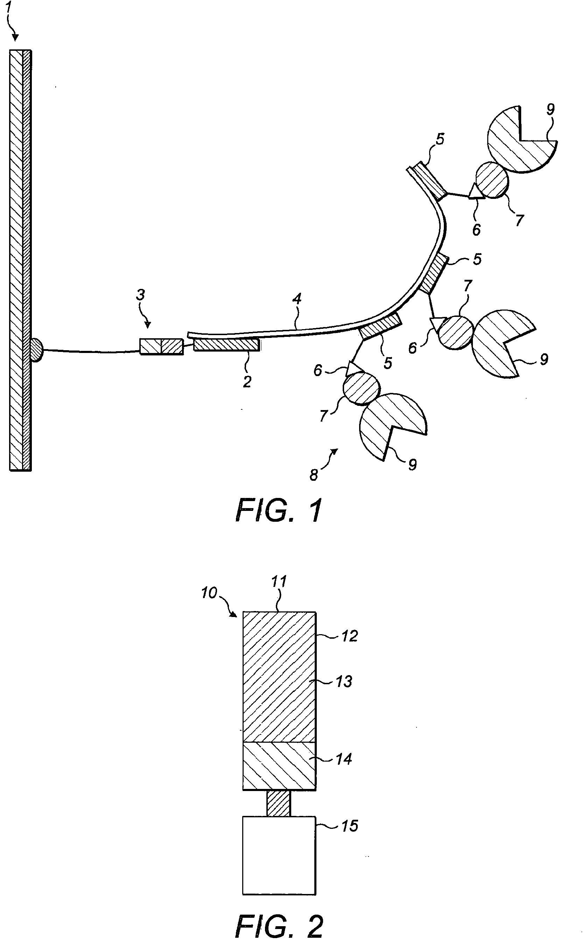

As shown in Figure 1, the invention provides an assay, having a working electrode 1, to which is attached a surface probe 2, via a linker 3.

The surface probe 2 comprises a known single stranded nucleic acid sequence, which is immobilised at the electrode by the linker arrangement The sulfo SMCC linker is however not the only method that we can utilise to covalently attach the surface probe to the surface of the electrode and several commercially available options are available. Non covalent bonding by simply drying the probe onto the electrode surface is also a possible solution.

Single stranded genomic DNA 4, from a sample to be tested, will bind the surface probe 2 if a sequence complementary to the surface probe sequence is present in the genomic DNA.

The assay comprises at least one further probe, being a signal probe 5. The signal probe 5 comprises a different nucleic acid sequence and is biotinylated.

These biotin tags 6 on the signal probe 5 will bind very strongly to a biotin binding protein 7 such as avidin or streptavidin.

Using the biotin-avidin (or streptavidin) system, an indicator molecule, such an enzyme that produces a physical change in the test solution, can be associated with the biotinylated signal probe 5.

In one embodiment of the invention, streptavidin-catalase dendrimers 8 are added to the test solution to bind to the biotin tags 6 on the signal probe.

Regardless of whether singular streptavidin or streptavidin dendrimers are used in the previous step, a biotinylated peroxidase is then introduced. This can be, for example, biotinylated HRP or biotinylated catalase.

Catalase 9 catalyses a reaction in the test solution to produce oxygen and water from hydrogen peroxide. This results in electrons being transferred to the measuring electrode, thus altering the charge.

One advantage of using catalase as the first catalytic element is that this produces a very fast reaction time. Catalase is one of the fastest enzymes. Some 40 million molecules of hydrogen peroxide can be converted to water and oxygen per second - generating some 20 million molecules of oxygen. Enzymes having a turnover rate of over 3000 molecules of substrate per second would be very useful in the invention as the catalyst.

### Sample preparation

As illustrated in Figure 2, the invention provides apparatus 10, having an opening 11, through which a sample may be introduced to a cell lysis chamber 12 containing cell lysis solution 13. In preparation of samples, biological samples are taken by swab or from liquid culture. The sample is placed into the lysis solution 13 appropriate for the sample and left for around 5 minutes.

Where the assay is being performed to test a sample for MRSA, swabs from a patient are placed into a solution containing lysostaphin and lysozyme for 3 minutes at 37°C. Lysostaphin and lysozyme are both enzymes that break down proteins found within the cell wall. These enzymes break down the cell walls of MRSA and allow separation of the organism's DNA from the rest of the cell contents. Separation and isolation of the DNA from the rest of the cell is required for efficient probe genomic DNA hybridisation.

The cell lysis chamber 12 is a thermostatically controlled chamber that allows for the extraction of genomic DNA from the cells in a sample. The DNA is then passed through a filter 14, which may comprise resin or beads to separate DNA from the rest of the sample contents.

The DNA is passed into a hybridisation chamber 15, where hybridisation to probes can occur.

A temperature control, linked to a power supply, is used to maintain the optimum temperature for cell lysis and hybridisation, in the cell lysis chamber 12 and hybridisation chamber 15 respectively.

The working electrode 1 is located in the hybridisation chamber 15, which may also have one or more pumps for passing solutions over the electrode surface.

A reference electrode is used in the apparatus in order to measure potential difference. A mechanical shield (not shown)may cover or expose the reference surface on the electrode. The mechanical shield covers the reference electrodes during the hybridisation phase preventing nonspecific binding of enzyme and providing cleaner results.

There may be further chambers as necessary for containing reaction solutions.

In a preferred embodiment the apparatus is an electronic device having a means for saving and exporting data (SSD drive and USB port).

### Methods of DNA isolation

### 1) Resin

The solution containing the lysed cells 13 may then be passed through a resin filter 14, which retains protein but allows lysed genomic DNA to pass through to a hybridisation (or testing) chamber.

### 2) DNA beads

The solution containing the lysed cells 13 may be passed through a column containing beads coated with a polymer that binds DNA non-specifically. The rest of the lyses cell debris passes into a waste chamber leaving the DNA bound to the beads. The DNA is then eluted off of the beads in a Tris solution (pH8) and passed into the hybridisation chamber.

### Hybridisation of biotinylated oligonucleotide probe to genomic DNA

With the lysed genomic DNA in the hybridisation chamber 15, hybridisation to both the surface and signal probes occurs in a Tris KCl hybridisation solution. The single stranded surface probe nucleic acid sequence binds to specific complementary sequences on the genomic DNA sequence. This fixes the genomic DNA to the surface of the electrode.

The biotinylated signal probe also binds to complementary sequences, which are further along the genome - and can be many kilobases away.

Genomic DNA may break into shorter strands during the cell lysis process, so by using multiple probes, the chances of fixing continuous strands of genomic DNA that feature both complementary surface and signal sequences are increased.

In the hybridisation chamber 15, genomic DNA is heated in the presence of both the signal probe and the electrode to 95°C for a brief time to separate the DNA strands.

The chamber is then cooled to a temperature appropriate to allow the probes to hybridise with the single stranded genomic DNA 4. At this point, the genomic DNA 4 is specifically bound to the electrode by its complementary sequence to the surface probe 2. Further along the genomic DNA strand 4, the biotinylated signal probe 5 is also attached to the genomic DNA 4.

A brief wash step is then applied to remove any unbound biotinylated signal probe 5 and prepare the working electrode 1 for the binding of the streptavidin- catalase dendrimers 8.

In order to prevent non-specific binding of streptavidin- catalase dendrimers 8 to the working electrode surface 1, a protein (not shown) is used to block the electrode. This is done by running a dialysis 0.5% casein solution over the surface of the electrode 1.

The casein adheres to the surface of the electrode 1, blocking any potential non-specific binding sites. Following application of the casein solution either or
- a streptavidin- catalase dendrimer 8
- a streptavidin HRP dendrimer
- singular streptavidin catalase
- singular streptavidin HRP casein solution,
is also run over the surface of the electrode. Instead of pre conjoining the Streptavidin and peroxidase it may be the case that streptavidin is applied first, followed by the biotinylated peroxidase. This will achieve the same end result as pre conjoining the streptavidin and peroxidase, but may be simpler to execute.

The streptavidin portion 7 of the streptavidin-peroxidase complex 8 binds to the biotin tags 6 on the signal probe 5, which is bound to the genomic DNA 4. This fixes the catalase 9 to the surface of the electrode 1 via the genomic DNA 4.

It is then necessary to wash the electrode surface by briefly running wash solution over it, to remove any non-specifically bound enzyme.

### Detection

### Using catalase

The presence or absence of catalase 9 is detected by flooding the chamber with H₂O₂ solution at a high pH. Catalase breaks down H₂O₂ and in the process, passes electrons to the surface of the working electrode 1.

### Using HRP

The presence or absence of HRP 9 is detected by flooding the chamber with TMB solution. HRP breaks down TMB and in the process, passes electrons to the surface of the working electrode 1. In either case, the peroxidase on the surface of the electrode in the presence of its substrate will result in a chemical reaction. This creates a charge difference (potential difference) between the working electrode 1 and a reference electrode, which is detected by the system. The total number of volts recorded across the electrode over a set time, indicates whether or not a specific pathogen is present in a sample.

### Examples (The Example using HRP as the catalyst instead of catalase does not form part of the claimed invention)

A luminol-based assay was performed, using HRP as the catalyst instead of catalase and detecting the assay stack using light HRP will only be present when the correct genomic DNA is present that allows the HRP to be bound to the surface of the electrode. By exposing the electrodes to a luminol solution and placing them in an imager we can detect the HRP on the surface of the electrode.

Two electrodes had MRSA specific surface probes covalently attached to their surfaces. Electrode 1 was exposed to a hybridization solution containing *E.Coli* genomic DNA and electrode 2 to MRSA DNA.

MRSA specific signal probes were also present in both hybridization solutions. In this case the signal and surface probes are separated by a distance of around 100kb. These were heated to 95 for 30 seconds then hybridized to the probe for 3 min at 45C.

The electrodes were then exposed to streptavidin-HRP before being imaged in the presence of luminol solution.

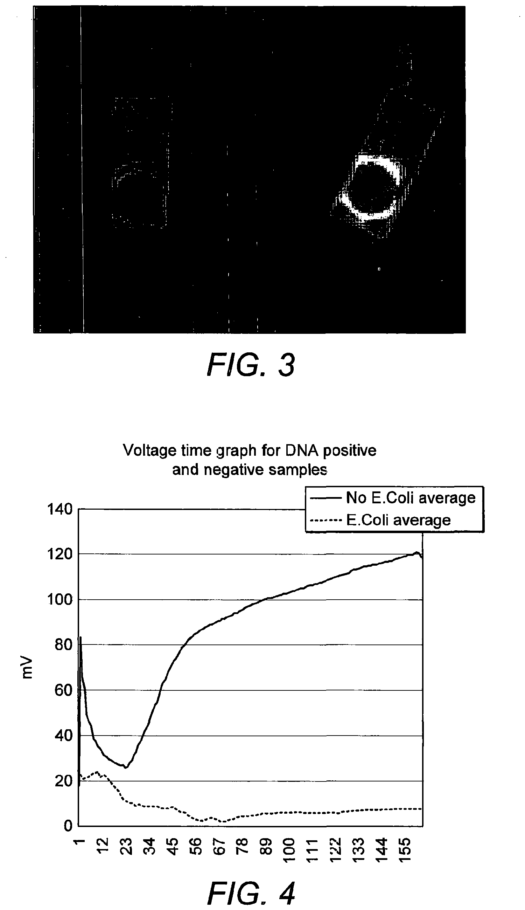

Figure 3 shows that the MRSA genomic DNA has bound to both surface and signal probes on electrode 2. This has not occurred on the E.Coli test and proves that the present system can be used to detect specific DNA sequences.

The detection of specific DNA sequences electronically via a potential difference is shown in the experimental results. The experiment used catalase as the enzyme. To demonstrate the detection of organisms other than MRSA, the test was performed using *E.coli* specific probes.

Instead of being analysed by the luminol light reaction the electrode was attached to a multimeter after the assay had been completed in the presence and absence of *E.coli* genomic DNA.

As shown in Figure 4, the electrodes that were exposed to *E.coli* genomic DNA produced a greater potential difference between the sensing electrode and the reference electrode. This was caused by the presence of catalase on the surface of the electrode, specifically tethered by the genomic DNA "bridge".

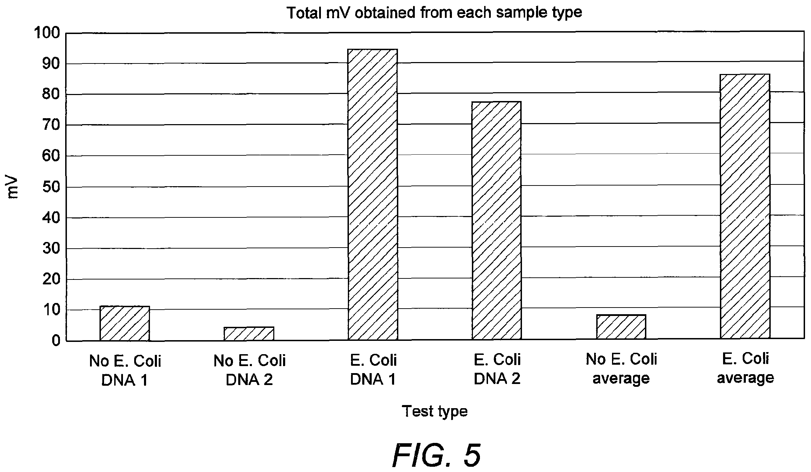

Figure 5 illustrates the total mV obtained over a 100 second period. As clearly illustrated in Figure 5, when in the presence of *E.coli* genomic DNA, catalase is linked to the surface of the electrode. When no genomic DNA is present, the DNA "bridge" cannot be completed and as such catalase cannot be tethered to the surface of the electrode.

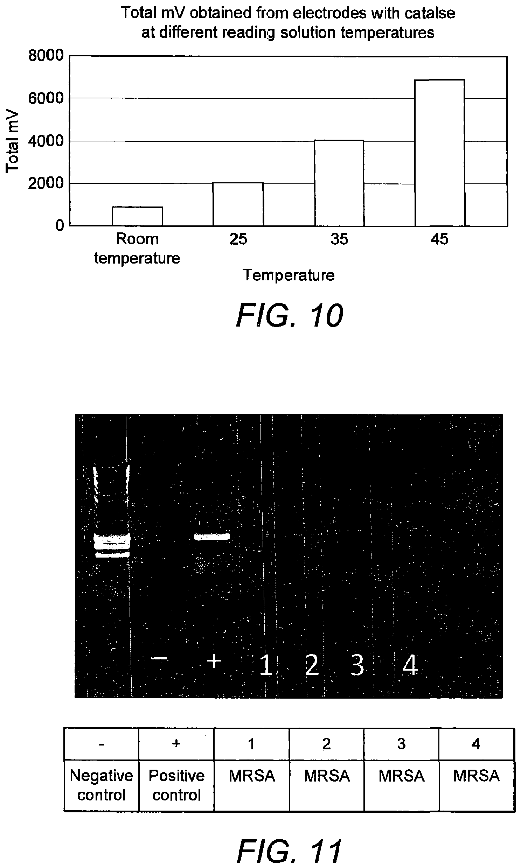

Figure 11 shows a PCR gel image taken of PCRs run with the lysed DNA from a rapid DNA extraction process. Gram positive MRSA cells were lysed using lysostaphin and lysozyme at 37°C. The process took five minutes to complete and was performed on approximately 10⁶ cells. The lysed DNA was tested with MRSA specific PCR primers to determine the presence of MRSA genetic material in the lysed sample.

Although specific embodiments of the invention refer to DNA analytes, it will be apparent to the skilled person the invention may be used to detect any nucleic acid analyte - such as RNA or cDNA.

One embodiment of the invention utilises multiple signal probes and/or multiple surface probes. Using different sequences, it is possible to include multiple signal probes, thus increasing the signal to genome ratio.

Similarly, multiple surface probes may be designed to attach to the genomic DNA at different locations, to ensure binding of the genomic DNA to the electrode surface.

### Example of surface and signal probe at distance greater than 12kb (not an Example of the claimed invention)

The ability to identify a specific DNA sequence using a pair of probes greater than 12kb apart on a chromosome provides a distinct advantage over existing pathogen detection systems.

To test this, we identified three MRSA-specific regions within the MRSA252 chromosome using the NCBI BLAST facility, these sequences were not found in either MSSA or *E. Coli.* One of the probes was chosen as a surface probe and so became the fixed point of origin from which the distances to the other probes was calculated. The sequence complementary to the first signal probe (EDS2) was shown to be approximately 12kb away from the sequence complementary to the surface probe on the chromosome, while a sequence complementary to a second signal probe (EDS41) was approximately 1.5 mega bases away.

Electrodes with surface probe bound to their surfaces were made and hybridised to either EDS2 or EDS41 in the presence of MRSA252 genomic DNA (equivalentto 10,000 cells). For a comparison, the tests were also performed without genomic DNA.

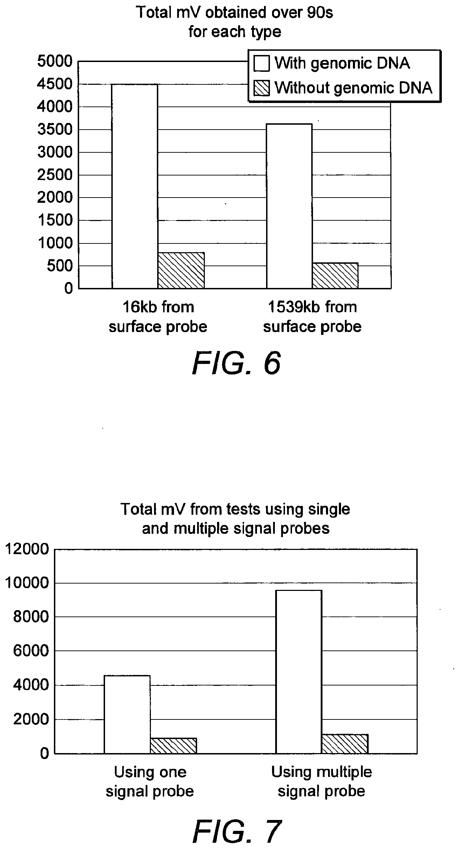

Hybridisation took place in a PCR tube with a 100µl reaction volume in Tris KCl buffer pH8. Following a 30 second denaturing step at 95°C hybridisation was performed at 45°C for 3 minutes. The electrodes were then washed to remove unbound signal probe, exposed to strep HRP before a second wash to remove unbound enzyme. The electrodes were then placed in TMB buffer and the potential difference generated recorded over 90 seconds. The sum of the potential differences was then calculated for each type and is shown in the graph in Figure 6. Figure 6 illustrates that the test easily detected the presence of the target MRSA252 genome in the sample medium using probes up to 1.5Mb apart. As the total length of the genome is only 2.8Mb, the invention, in this case, represents a method of identifying an organism using probes targeted at sequences at opposite ends of the pathogen's chromosome.

### Example of multiple probe use (not an Example of the claimed invention)

The ability to identify a specific DNA sequence using more than two probes provides the invention with a distinct advantage over existing pathogen detection systems. To test this, eight MRSA specific regions were identified within the MRSA252 chromosome using the NCBI BLAST facility. These sequences were not found in either MSSA or *E. Coli.* One of these probes (EDS3) was chosen as a surface probe while the rest were chosen as signal probes.

Electrodes with surface probe bound to their surfaces were constructed and bridged by genomic DNAto either one probe (EDS2) or all eight probes (EDS2, EDS41, EDS42, EDS43, EDS44, EDS425a, EDS425b, EDS) in the presence of MRSA252 genomic DNA equivalent to 10,000 cells. For a comparison the tests were also performed without genomic DNA.

Hybridisation took place in a PCR tube with a 100µl reaction volume in Tris KCl buffer pH8. Following a 30 second denaturing step at 95°C hybridisation was performed at 45°C for 3 minutes. The electrodes were then washed to remove unbound signal probe, exposed to strep HRP before a second wash to remove unbound enzyme. The electrodes were then placed in TMB buffer and the potential difference generated recorded over 90 seconds. The sum of the potential differences was then calculated for each type and is shown in the graph in Fig 7.

The results of the experiment shown in Fig 7 indicate that by using multiple probes the sensitivity of the test is increased.

### Increased catalase signal by altering pH

The performance of catalase as a potentiometric signalling enzyme in our assay can be increased significantly by altering the pH and temperature of the solution which it is used to detect the enzyme. Catalase in free solution breaks down H₂O₂ to H₂O at an extremely fast rate in cyclic reaction. The first breakdown of H₂O₂ will result in the release of an electron that is normally used to break down the following H₂O₂ molecule that enters the reaction site. When the catalase, in the presence of H₂O₂, is attached (albeit indirectly) to the surface of an electrode, the electron may be passed onto the electrode or the catalase may take electrons from the electrode during the enzymatic reaction. This is what creates the potential difference that is detected by the electrode reader.

Commonly, catalase is used at a pH of around 7. However, it has been discovered that by increasing or decreasing the pH of the solution in which the electrode is read, electron donation may be driven in a specific direction. At pH 7 the ionic potential of the system is balanced, allowing the catalase to either accept or donate electrons from the electrode. At higher or lower pH, the catalase is forced to either donate or accept electrons from the electrode - resulting in higher potential difference generation from the same amount of catalase.

To illustrate this, three electrodes had 2µL of 0.1mg/ml catalase dried onto their working surfaces. This was achieved by placing the electrodes in a 37°C incubator for 1 hour. 100mM Tris solutions were made up at pH 5, 7 and 9 before the addition of H₂O₂ to a concentration of 0.3%.

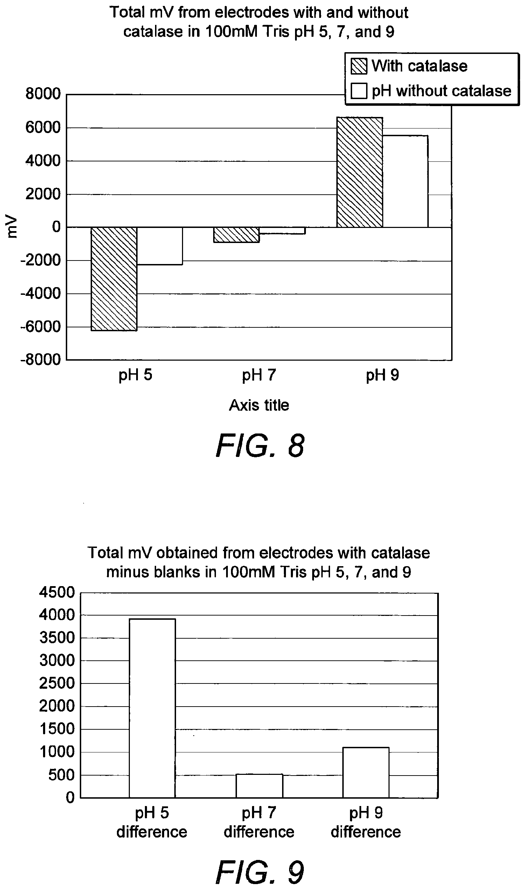

The electrodes were placed into the reading solutions and the potential difference read for 90 seconds. For reference, blank electrodes (which did not have catalase dried onto them) were placed in equivalent solutions. The sum of the total voltage obtained from each type was calculated and is shown in Fig 8.

There was a shift from a negative potential difference to a positive one as the pH increases, which reflects how the ionic balance of the reading solution is causing the catalase to either accept or donate electrons to the working electrode.

The total mV blank values for each type was subtracted from the total mV obtained from electrodes with catalase to provide the voltage generated from the catalase in each reading solution. The results are shown in Figure 9.

Fig 9 shows that by altering the pH away from 7 in either direction, the catalase produces a greater potential difference than when tested in a reading solution that is pH7.

### Increased catalase signal by increasing temperature

It has further been discovered that the signal obtained from catalase may be amplified by increasing the temperature of the reading solution. This increases the rate of H₂O₂ breakdown, electron transfer, and total potential difference.

To illustrate this,, four electrodes had 4uL of 1.0 mg/ml catalase dried onto their working surfaces. This was achieved by placing the electrodes in a 37°C incubator for 1 hour.

The electrodes were placed into the reading solution (50mM Tris pH 7) at room temperature, 25°C, 35°C and 45°C. The potential difference was recorded at 1 second intervals for 90 seconds. The sum of the total voltage obtained from each type was calculated and is shown in Fig 10. Fig 10 shows that by increasing the temperature of the reading solution the electronic signal obtained by catalase attached to the electrode can be dramatically increased.

### Multiplex methods

The assay can be used to identify either a single or multiple species in a sample via a species-specific sequence. The assay can be configured to identify whether any DNA from a known group of species is present in the sample or identify which known species are present.

Any specified species from a sample can be identified utilising a single working electrode.

Target species are predefined prior to testing and dictate the types of surface probes bound to the surface and signal probes used. This type of testing may be useful in the environmental sector, for example where a user would wish to determine whether a water source contains any one of a number of dangerous bacterial species.

Unique sequences found in each of the species that the user wishes to detect would be identified by genetic analysis and selected. From these unique sequences, surface and signal probes would be produced for use in the test. A mix of surface probes pertaining to each species that is to be tested, are created and attached to the surface of the electrode.

A sample (eg river water) is collected and placed in the lysis solution to release pathogen DNA.

The test is then run using this extracted DNA, which will hybridise to both the surface probes on the electrode and the signal probes in the reaction mixture. In this case, the presence of any of the bacteria in the river water that the test has been set up to detect will result in a positive signal and reveal that the water poses a health risk. Thus, the assay identifies the presence of one or more of a number of pathogens in a sample.

Using the same technology but with a slightly different configuration, the assay can separately identify specific organisms from a single sample. This is slightly more sophisticated and utilises multiple electrodes. This type of testing may be useful in the food industry - for example where a user would wish to determine whether a meat product contains tissue originating from species other than that claimed by the manufacturer. For example, a user may wish to check that a beef burger is 100% beef. A quick way to determine this is to check that there are no DNA sequences present in the product unique to species commonly used as cheaper replacements to beef.

Unique sequences found in each of the species suspected of being in the beef burger (other than cows) are identified by genetic analysis. From these unique sequences, surface and signal probes are produced and used in the test. Several electrodes are used in a single test - each coated with surface probes originating from the genome of a different species. By doing this, species-specific electrodes are created within the electrode series e.g. horse DNA only electrode, pig DNA only electrode etc. The test is run on a sample of DNA extracted from the burger, which is hybridised in the presence of the electrodes in a solution containing the signal probes from all of the species. Because of the specificity of the surface probe(s), only a specific species can bind to its appropriate electrode, each electrode when read will therefore produce a signal for each species found in the sample.

The skilled person will appreciate that the test is not restricted to using this specific methodology and that the assay may also be arranged to determine the presence of sub species by identifying sequences unique to each type. This may be useful, for example, in differentiating between virulent and non-virulent wild types.

### Rapid DNA sensing development strategy

The ability to rapidly develop and deliver DNA sensing kits for novel pathogens anywhere in the world potentially has enormous ramifications for disease control. The assay uses nucleic acid sensing technology, which may provide health authorities or private customers close to the outbreak zone with sensitive, rapid portable kits designed to detect the pathogen they are looking for, whether it be bacterial or viral. The stages of providing a test in response to a specific need or novel pathogen are outlined below:

### Stage 1 Analysis of novel pathogen

Once a novel pathogen has been identified a sample of it will be provided for whole genome analysis. Upon receipt of the sample the pathogen will be characterised as RNA virus, DNA virus, gram positive, gram negative or fungal. A suitable lysis method will then be used to obtain genomic DNA from the pathogen in question (in the case of RNA viruses a reverse transcription step may be required to convert the RNA into DNA). This will take around 1 day to achieve,

### Stage 2 Whole genome sequencing

Genomic DNA will then be sequenced by a whole genome sequencing approach to define the full genetic code of the pathogen. This process will take up to 5 days to complete.

### Stage 3 Isolation of suitable DNA sequences for probes

The genetic code of the pathogen will then be analysed for suitable unique sequences that will be used to identify the pathogen in question. At least two sequences will be identified during this process. This will take around 2 days to complete.

### Stage 4 Production of DNA sensing probes

The unique sequences identified in the previous step will then be sent to an oligonucleotide production company for production. The sequences will be either thiolated to be used as surface probes in the assay or biotinylated for use as sensing probes. This process of up to 2 days to complete.

### Stage 5 Production of kit

The assay electrodes will be assembled and the standard core assay kit altered to detect the pathogen of choice. Firstly this will involve putting in place a DNA extraction method suitable for the pathogen of choice - i.e. a gram negative cartridge, viral cartridge etc. Secondly a cartridge containing the biotinylated probes will be added into the assay system. These two components, along with the pathogen specific electrode will allow identification of the pathogen in question. This process will take around 2 days to complete. If necessary, a trial will then be set up to determine the L.O.D and specificity of the test the pathogen in question. This will take around 2 days to complete depending on the number of samples. Larger scale trials may not be necessary if similar work has been carried out on a similar organism.

### Stage 6 Delivery of kit

Once confirmed as effective, the kit can then be mass produced, using in house printing techniques, many thousands of kits may be manufactured within a week. These can then be shipped to any location on the planet within 1 day.

## Claims

1. An assay for detecting an analyte in a biological sample comprising:
at least one surface probe linked to the surface of a measuring electrode,
the surface probe comprising a nucleic acid sequence complementary to a first target nucleic acid sequence;
at least one signal probe comprising a nucleic acid sequence complementary to a second target nucleic acid sequence;
and wherein the at least one signal probe comprises a binding element capable of binding a ligand,
the ligand being associated with a catalytic element or precursor thereof capable of reacting with a substrate to alter electrical potential of the measuring electrode, wherein the catalytic element comprises catalase, and optionally the substrate comprises hydrogen peroxide; and a reference electrode against which a potential difference between the electrodes is measured wherein the assay further comprises potentiometric measuring apparatus comprising the measuring electrode and the reference electrode.

2. An assay as claimed in Claim 1, wherein the at least one signal probe is substantially free in a test solution when in an unhybridised state.

3. An assay as claimed in Claim 1 or 2, wherein the at least one signal probe is immobilised by hybridisation to the second target nucleic acid sequence of a single stranded nucleic acid that also comprises the first target nucleic acid sequence;
and optionally, wherein the at least one signal probe is immobilised by the single stranded nucleic acid tethering the at least one signal probe to the at least one surface probe.

4. An assay as claimed in any of Claims 1 to 3, wherein the at least one signal probe comprises a biotinylated oligonucleotide.

5. An assay as claimed in any preceding claim, wherein the binding element comprises biotin.

6. An assay as claimed in claim 1, wherein the ligand comprises avidin or streptavidin.

7. An assay as claimed in any preceding claim, comprising a single hybridisation cycle.

8. An assay as claimed in any preceding claim, wherein the distance between the first target nucleic acid sequence and the second target nucleic acid sequence is greater than 12kB.

9. An assay as claimed in any preceding claim, wherein the assay comprises a plurality of surface probes, signal probes and/or measuring electrodes.

10. A method for detecting an analyte comprising the steps of: providing a single stranded nucleic acid from a sample;
exposing the single stranded nucleic acid to at least one surface probe and at least one signal probe under hybridisation conditions;
wherein the at least one surface probe is linked to the surface of a measuring electrode of a potentiometric measuring apparatus, and wherein the at least one signal probe comprises a binding element capable of binding a ligand;
providing a ligand and a catalytic element associated with the ligand,
wherein the catalytic element comprises catalase;
providing a substrate for the catalytic element;
detecting the presence or absence of an analyte by measuring a potential difference between the measuring electrode and a reference electrode of the potentiometric measuring apparatus.

11. A method as claimed in Claim 10, wherein the at least one signal probe is substantially free in a test solution when in an unhybridised state.

12. A method as claimed in Claim 10 or 11, wherein the at least one signal probe is immobilised by hybridisation to the second target nucleic acid sequence when the single stranded nucleic acid from the sample also comprises the first target nucleic acid sequence.

13. A method as claimed in any of Claims 10 to 12, further comprising at least one washing step, and optionally comprising at least one blocking step, and optionally comprising the step of increasing the temperature to above about 25°C.

14. A method as claimed in Claim 10, comprising the step of adjusting the pH to above or below pH 7.

## Patentansprüche

1. Ein Assay zum Detektieren eines Analyten in einer biologischen Probe, beinhaltend:
mindestens eine Oberflächensonde, die mit der Oberfläche einer Messelektrode verbunden ist, wobei die Oberflächensonde eine zu einer ersten Zielnukleinsäuresequenz komplementäre Nukleinsäuresequenz beinhaltet;
mindestens eine Signalsonde, die eine zu einer zweiten Zielnukleinsäuresequenz komplementäre Nukleinsäuresequenz beinhaltet;
und wobei die mindestens eine Signalsonde ein Bindungselement beinhaltet, das in der Lage ist, einen Liganden zu binden, wobei der Ligand mit einem katalytischen Element oder Präkursor davon assoziiert ist, das/der in der Lage ist, mit einem Substrat zu reagieren, um das elektrische Potential der Messelektrode zu ändern, wobei das katalytische Element Katalase beinhaltet und wobei optional das Substrat Wasserstoffperoxid beinhaltet; und
eine Referenzelektrode, gegen die eine Potentialdifferenz zwischen den Elektroden gemessen wird,
wobei das Assay ferner eine potentiometrische Messvorrichtung, beinhaltend die Messelektrode und die Referenzelektrode, beinhaltet.

2. Assay gemäß Anspruch 1, wobei die mindestens eine Signalsonde in einer Testlösung im Wesentlichen frei ist, wenn sie sich in einem nicht hybridisierten Zustand befindet.

3. Assay gemäß Anspruch 1 oder 2, wobei die mindestens eine Signalsonde durch Hybridisierung an die zweite Zielnukleinsäuresequenz einer einzelsträngigen Nukleinsäure, die auch die erste Zielnukleinsäuresequenz beinhaltet, immobilisiert wird; und optional, wobei die mindestens eine Signalsonde durch die einzelsträngige Nukleinsäure immobilisiert wird, die die mindestens eine Signalsonde an die mindestens eine Oberflächensonde anbindet.

4. Assay gemäß einem der Ansprüche 1 bis 3, wobei die mindestens eine Signalsonde ein biotinyliertes Oligonukleotid beinhaltet.

5. Assay gemäß einem der vorhergehenden Ansprüche, wobei das Bindungselement Biotin beinhaltet.

6. Assay gemäß Anspruch 1, wobei der Ligand Avidin oder Streptavidin beinhaltet.

7. Assay gemäß einem der vorhergehenden Ansprüche, beinhaltend einen einzelnen

8. Assay gemäß einem der vorhergehenden Ansprüche, wobei der Abstand zwischen der ersten Zielnukleinsäuresequenz und der zweiten Zielnukleinsäuresequenz mehr als 12 kB beträgt.

9. Assay gemäß einem der vorhergehenden Ansprüche, wobei das Assay eine Vielzahl von Oberflächensonden, Signalsonden und/oder Messelektroden beinhaltet.

10. Ein Verfahren zum Detektieren eines Analyten, beinhaltend die folgenden Schritte:
Bereitstellen einer einzelsträngigen Nukleinsäure aus einer Probe;
Aussetzen der einzelsträngigen Nukleinsäure gegenüber mindestens einer Oberflächensonde und mindestens einer Signalsonde unter Hybridisierungsbedingungen;
wobei die mindestens eine Oberflächensonde mit der Oberfläche einer Messelektrode einer potentiometrischen Messvorrichtung verbunden ist und wobei die mindestens eine Signalsonde ein Bindungselement beinhaltet, das in der Lage ist, einen Liganden zu binden;
Bereitstellen eines Liganden und eines katalytischen Elements, das mit dem Liganden assoziiert ist, wobei das katalytische Element Katalase beinhaltet;
Bereitstellen eines Substrats für das katalytische Element;
Detektieren des Vorhandenseins oder des Fehlens eines Analyten durch Messen einer Potentialdifferenz zwischen der Messelektrode und einer Referenzelektrode der potentiometrischen Messvorrichtung.

11. Verfahren gemäß Anspruch 10, wobei die mindestens eine Signalsonde in einer Testlösung im Wesentlichen frei ist, wenn sie sich in einem nicht hybridisierten Zustand befindet.

12. Verfahren gemäß Anspruch 10 oder 11, wobei die mindestens eine Signalsonde durch Hybridisierung an die zweite Zielnukleinsäuresequenz immobilisiert wird, wenn die einzelsträngige Nukleinsäure aus der Probe auch die erste Zielnukleinsäuresequenz beinhaltet.

13. Verfahren gemäß einem der Ansprüche 10 bis 12, ferner beinhaltend mindestens einen Waschschritt und optional beinhaltend mindestens einen Blockierschritt und optional beinhaltend den Schritt des Erhöhens der Temperatur auf über etwa 25 °C.

14. Verfahren gemäß Anspruch 10, beinhaltend den Schritt des Anpassens des pH-Werts auf über oder unter einen pH-Wert von 7.

## Revendications

1. Un dosage pour la détection d'un analyte dans un échantillon biologique comprenant :
au moins une sonde de surface reliée à la surface d'une électrode de mesure, la sonde de surface comprenant une séquence d'acide nucléique complémentaire à une première séquence d'acide nucléique cible ;
au moins une sonde de signal comprenant une séquence d'acide nucléique complémentaire à une deuxième séquence d'acide nucléique cible ;
et dans lequel l'au moins une sonde de signal comprend un élément de liaison capable de lier un ligand, le ligand étant associé à un élément catalytique ou un précurseur de celui-ci capable de réagir avec un substrat afin de modifier le potentiel électrique de l'électrode de mesure, dans lequel l'élément catalytique comprend une catalase, et facultativement le substrat comprend un peroxyde d'hydrogène ; et
une électrode de référence par rapport à laquelle est mesurée une différence de potentiel entre les électrodes,
le dosage comprenant en sus un appareil de mesure potentiométrique comprenant l'électrode de mesure et l'électrode de référence.

2. Un dosage tel que revendiqué dans la revendication 1, dans lequel l'au moins une sonde de signal est substantiellement libre dans une solution d'essai lorsqu'elle est dans un état non hybridé.

3. Un dosage tel que revendiqué dans la revendication 1 ou la revendication 2, dans lequel l'au moins une sonde de signal est immobilisée par hybridation à la deuxième séquence d'acide nucléique cible d'un acide nucléique simple brin qui comprend aussi la première séquence d'acide nucléique cible ;
et facultativement, dans lequel l'au moins une sonde de signal est immobilisée par l'acide nucléique simple brin ancrant l'au moins une sonde de signal à l'au moins une sonde de surface.

4. Un dosage tel que revendiqué dans n'importe lesquelles des revendications 1 à 3, dans lequel l'au moins une sonde de signal comprend un oligonucléotide biotinylé.

5. Un dosage tel que revendiqué dans n'importe quelle revendication précédente, dans lequel l'élément de liaison comprend la biotine.

6. Un dosage tel que revendiqué dans la revendication 1, dans lequel le ligand comprend l'avidine ou la streptavidine.

7. Un dosage tel que revendiqué dans n'importe quelle revendication précédente, comprenant un seul cycle d'hybridation.

8. Un dosage tel que revendiqué dans n'importe quelle revendication précédente, dans lequel la distance entre la première séquence d'acide nucléique cible et la deuxième séquence d'acide nucléique cible est de plus de 12 kB.

9. Un dosage tel que revendiqué dans n'importe quelle revendication précédente, le dosage comprenant une pluralité de sondes de surface, de sondes de signal et/ou d'électrodes de mesure.

10. Un procédé pour la détection d'un analyte comprenant les étapes consistant à :
mettre à disposition un acide nucléique simple brin provenant d'un échantillon ;
exposer l'acide nucléique simple brin à au moins une sonde de surface et à au moins une sonde de signal dans des conditions d'hybridation ;
dans lequel l'au moins une sonde de surface est reliée à la surface d'une électrode de mesure d'un appareil de mesure potentiométrique, et dans lequel l'au moins une sonde de signal comprend un élément de liaison capable de lier un ligand ;
mettre à disposition un ligand et un élément catalytique associé au ligand, l'élément catalytique consistant en une catalase ;
mettre à disposition un substrat pour l'élément catalytique ;
détecter la présence ou l'absence d'un analyte en mesurant une différence de potentiel entre l'électrode de mesure et une électrode de référence de l'appareil de mesure potentiométrique.

11. Un procédé tel que revendiqué dans la revendication 10, dans lequel l'au moins une sonde de signal est substantiellement libre dans une solution d'essai lorsqu'elle est dans un état non hybridé.

12. Un procédé tel que revendiqué dans la revendication 10 ou la revendication 11, dans lequel l'au moins une sonde de signal est immobilisée par hybridation à la deuxième séquence d'acide nucléique cible lorsque l'acide nucléique simple brin provenant de l'échantillon comprend aussi la première séquence d'acide nucléique cible.

13. Un procédé tel que revendiqué dans n'importe lesquelles des revendications 10 à 12, comprenant en sus au moins une étape de lavage, et comprenant facultativement au moins une étape de blocage, et comprenant facultativement l'étape consistant à augmenter la température jusqu'à au-dessus de 25 °C environ.

14. Un procédé tel que revendiqué dans la revendication 10, comprenant l'étape consistant à régler le pH jusqu'à au-dessus ou au-dessous de pH 7.
